# EUROPÄISCHE PATENTANMELDUNG

(11) **EP 1 862 164 A2**
(43) Veröffentlichungstag der Anmeldung: **05.12.2007**
(21) Anmeldenummer: 07116946.0
(22) Anmeldetag: 10.11.1999
(51) Int. Cl.: A61K 9/72, A61K 47/12

(54) **Trockenpulver zur Inhalation**

(30) Priorität: 13.11.1998 CH 228698
(62) Teilanmeldung aus: 02025796.0
(71) Anmelder: Jagotec AG, 4132 Mutenz (CH)
(72) Erfinder: Keller, Manfred, 79189, Bad Krozingen (DE); Müller-Walz, Rudi, 79650, Schopfheim (DE)
(74) Vertreter: Hoechst, Bruno Werner

(57) **Zusammenfassung**

Trockenpulver-Formulierung zur Inhalation, die ein Pulvergemisch enthaltend einen pharmazeutisch nicht wirksamen Träger in nicht inhalierbarer Teilchengrösse, einen fein verteilten pharmazeutischen Wirkstoff in inhalierbarer Teilchengrösse und staubförmiges Magnesiumstearat einer mittleren Teilchengrösse von 1 bis 100 µm umfasst.

## Beschreibung

Die Erfindung betrifft die Verbesserung der Feuchtigkeitsbeständigkeit von Trockenpulver-Formulierungen zur Inhalation sowie die neue Trockenpulver-Formulierungen.

Trockenpulver-Formulierungen zum Inhalieren müssen eine Reihe von einander teilweise widersprechenden Anforderungen erfüllen, wobei insbesondere die folgenden zu beachten sind:
Der Wirkstoff muss inhalierbar sein. Um in die Lunge gelangen zu können, muss er in Partikeln von ca. 1 bis 10 µm Grösse vorliegen. Solche mikrofeinen Partikel können beispielsweise durch Mikronisierung, kontrollierte Ausfällung aus geeigneten Solventien oder durch Sprühtrocknung erzielt werden, wenn die Verfahrensbedingungen geeignet gewählt, kontrolliert und ausgeführt werden. Mikrofeine Partikel haben jedoch ein sehr ungünstiges, d.h. grosses Verhältnis von Oberfläche zu Volumen bzw. Masse und daher eine grosse Oberflächenenergie. Dies äussert sich in starken Adhäsions-und Kohäsionstendenzen, die wiederum zu schlechten Fliesseigenschaften und zu Pulveraggregation führen. Derartige mikrofeine Pulver sind deshalb schwer zu handhaben und werden stark beeinflusst durch elektrostatische Aufladung, Bearbeitung, Luftfeuchtigkeit und dergleichen.

Um eine konsistente Herstellung der Formulierung, eine maschinelle Befüllung des Pulverinhalators und korrektes Dosieren und Freisetzen durch den Pulverinhalator zu gewährleisten, muss das Pulver frei fliessend sein. Gute Fliesseigenschaften erwartet man in der Regel bei genügend grossen, möglichst kugelförmigen Partikeln, die eine geringe Oberflächenenergie und kleine Kontaktflächen haben.

Bei Pulverinhalatoren mit einem Reservoir wird die fertige Arzneizubereitung in Form eines Pulverbetts in das Vorratsgefäss gefüllt. Eine Dosis wird durch eine geeignet ausgebildete Dosiervorrichtung entnommen. Die Entnahme erfolgt volumetrisch. Das genaue volumetrische Dosieren der Zubereitung erfordert für die meisten Wirkstoffe eine Verdünnung derselben mit einem pharmazeutisch inaktiven Hilfsstoff, um eine den Anforderungen an die Dosiergenauigkeit genügende, dosierbare Einheitsmenge zu erhalten.

Für Pulverinhalatoren, die das Medikament aus vordosierten Einheiten, z.B. Kapseln oder Blistern, freisetzen, gilt die gleiche Einschränkung für den reibungslosen Betrieb der Befüllungsmaschinen dieser Einheitsdosen.

Bei einem Multidosis-Trockenpulverinhalator, der ein Pulverreservoir enthält, aus dem die einzelnen Dosen über einen Dosiermechanismus entnommen werden, ist das pulverförmige Medikament in der Regel in Kontakt mit der Umgebungsluft und kann so durch Luftfeuchtigkeit beeinflusst werden. Die Qualität des Medikaments und des Inhalationssystems darf sich jedoch durch den Einfluss von äusseren Faktoren während der vorgesehenen Lagerzeit und bis zum Aufbrauchen der Packung nicht wesentlich verschlechtern.

Um diesen Anforderungen zu genügen, werden die inhalierbaren, d.h. in mikrofeinen Partikeln vorliegenden Bestandteile (Wirkstoffe) mit pharmakologisch inaktiven Substanzen gemischt, um fliessfähige Pulver zu erhalten. Die Verdünnung wird dabei so gewählt, dass die vom Pulverinhalator ausgebrachte Menge genau die gewünschte Dosis enthält. Der überwiegende Anteil des pharmakologisch inaktiven Hilfsstoffes liegt dabei absichtlich in einer Partikelgrösse vor, die nicht inhalierbar ist. Er dient nicht nur zum Verdünnen, sondern auch zum Einstellen einer akzeptablen, möglichst einer guten bis sehr guten Fliessfähigkeit der Pulvermischung. Er ist im Fall dieser sogenannten interaktiven oder geordneten Mischungen die Trägersubstanz, an die die mikrofeinen Wirkstoffpartikel durch Adhäsion gebunden werden, um so eine geeignete Mischgüte, d.h. Homogenität der Mischung, zu erreichen und aufrecht zu erhalten. Durch das Mischverfahren kann sich die Partikelgrösse des Trägers auch so ändern, dass ein bestimmter Anteil inhalierbar wird. Die Partikelgrösse des eingesetzten Trägers richtet sich dabei in der Regel nach den Anforderungen und Gegebenheiten des Pulverinhalators, der für die Applikation der Formulierung vorgesehen ist. Für diese Mischungen gilt, dass während allen notwendigen Verarbeitungs-, Transport-, Lager- und Dosiervorgängen keine Entmischung stattfinden darf, d.h. die Wirkstoffpartikel sich nicht von ihren Trägerteilchen ablösen dürfen. Während dem Dispergieren im Inhalator, ausgelöst durch den Atemfluss des Patienten, müssen die Wirkstoffpartikel aber möglichst effektiv, d.h. möglichst quantitativ, abgelöst werden, um inhaliert werden zu können. Der Träger (Carrier) ist in den meisten Fällen Lactose, kann aber auch Mannitol, Trehalose oder ein anderes geeignetes Trägermaterial sein. In manchen auf dem Markt erhältlichen Inhalatoren ist auch Glucose als Trägermaterial enthalten.

Es ist bekannt, dass die Fliesseigenschaften geordneter Mischungen in der Hauptsache von den physikalisch-chemischen Eigenschaften des Trägers, der ja in der Regel im Überschuss beigemischt wird, abhängen. Ebenso ist bekannt, dass die Effektivität der Freisetzung der inhalierbaren Primärpartikel des Wirkstoffs durch Scherkraft neben den physikalisch-chemischen, stoffspezifischen Eigenschaften des Wirkstoffes und den physikalischen, insbesondere aerodynamischen Eigenschaften des Pulverinhalators vor allem auch von den Eigenschaften des Trägers abhängt. Als analytische Messgrösse wird dazu in vitro in sogenannten Kaskadenimpaktoren oder Liquid Impingern, wie sie in verschiedenen Pharmakopöen beschrieben sind, die Menge Wirkstoff in feinen, inhalierbaren Partikeln (Feinpartikeldosis bzw. Fine Particle Dose, nachfolgend auch mit FPD bezeichnet) bzw. der Feinpartikelanteil (Fine Particle Fraction, nachfolgend auch mit FPF bezeichnet) bezogen auf die Gesamtmenge an abgegebenem Wirkstoff bestimmt.

Kürzliche Arbeiten zeigen, dass die FPF umso höher ist, je kleiner die Partikelgrösse der zugemischten Lactose ist [M.J. Clarke, U.J. Potter, P. Lucas, M.J. Tobyn und J.N. Staniforth: Posterpräsentation auf der Konferenz "Drug Delivery to the Lungs VIII" der Aerosol Society, London, 15.-16.12.1997; und P. Lucas, M.J. Clarke, K. Anderson, M.J. Tobyn und J.N. Staniforth (1998): Vortrag auf der Konferenz "Respiratory Drug Delivery VI", Hilton Head Island, 3.-7.5.1998, veröffentlicht in: R.N. Dalby, P.R. Byron und S.J. Farr (Herausgeber): Respiratory Drug Delivery VI, Interpharm Press, 1998, 243 ff.]. Dieses Verfahren stösst jedoch an eine natürliche Grenze, da die Fliessfähigkeit mit kleineren Partikeln rasch unzureichend wird.

Es konnte ebenfalls gezeigt werden, dass beim Vergleich gleicher Siebfraktionen von verschiedenen Lactosequalitäten eine umkristallisierte Lactose die höhere FPF erzielte [N.M. Kassem und D. Ganderton: J. Pharm. Pharmacol. 42 (1990), 11 ff. (Suppl.) und EP-B-0 464 171]. Dieser Effekt beruht auf der Tatsache, dass die Wirkstoffpartikel bevorzugt an Fehlstellen, Risse und Brüche, d.h. an besonders aktivierte Zentren (sogenannte "active sites" oder "hot spots") der Trägerpartikel anhaften. Die Adhäsionskräfte sind an diesen aktivierten Zentren am grössten und damit auch die Ablösung während der Inhalation am wenigsten wahrscheinlich. Es konnte nun durch elektronenmikroskopische Aufnahmen gezeigt werden, dass die umkristallsierte Lactose sehr viel regelmässiger ist als die handelsübliche Ware.

Weiterhin ist bekannt, dass auch kristallines α-Lactose-Monohydrat einen geringen Anteil amorphe Lactose enthält, die die regelmässige Kristallstruktur stört und damit für aktivierte Stellen auf der Kristalloberfläche sorgt [G. Buckton und P. Darcy: Int. J. Pharm. 123 (1995), 265 ff.; E.M. Phillips: Int. J. Pharm. 149 (1997), 267 ff.]. Wasser kann bei erhöhter Luftfeuchtigkeit bevorzugt an diese amorphen Zentren anlagern und als Weichmacher eine Umwandlung in die thermodynamisch stabilere Kristallform verursachen [B.C. Hancock und G. Zografi: J. Pharm. Sci. 86 (1997), 1 ff.]. Das wiederum hat zur Folge, dass die Lagerstabilität derartiger Pulverzubereitungen bei erhöhter Luftfeuchte begrenzt ist.

In WO-A-95/11666 wurde vorgeschlagen, die aktiven Zentren durch Zusatz von mikrofeiner Lactose abzusättigen mit dem Ziel, dem Wirkstoff beim Herstellen der endgültigen Mischung nur noch weniger energiereiche Bindungsstellen auf der Lactose zur Verfügung zu stellen. Da die Ablösung während der Inhalation demzufolge weniger Energie benötigt, sollte die FPF signifikant steigen, was eindeutig nachgewiesen werden konnte. Gleiches gilt auch für das Verfahren, das in WO-A-93/11746 beschrieben ist.

In J. Pharm. Pharmacol. 34: 141-145 (1982) wurde ferner gefunden, dass der Zusatz einer dritten Pulverkomponente zu einer vorher gebildeten geordneten Mischung aus Salicylsäure (1%) und Sucrose infolge Ladungswechselwirkungen die physikalische Stabiltät von ternären Mischungen in unterschiedlicher Weise beeinflussen kann. Die Zugabe von 0,5-4,0% Magnesiumstearat beeinträchtigte die Adhäsion der Salicylsäure-Partikel an den Sucrose-Träger, wobei sich der Anteil schwach gebundener Wirkstoffpartikel mit zunehmender Magnesiumstearat-Konzentration erhöhte. Dieser Befund wurde einer Änderung der Ladungswechselwirkungen auf der Oberfläche der Sucrose-Trägerpartikel als Folge der positiven elektrostatischen Ladung des Magnesiumstearats und der negativen Ladung der Salicylsäure- und Sucrose-Partikel zugeschrieben. Auf diesen Effekt und die Tatsache, dass die Zugabe einer dritten Komponente, die bevorzugt an den Trägerpartikeln anlagert, die Wirkstoffpartikel von ihren Adhäsionsstellen verdrängen kann, wurde bereits in J. Pharm. Pharmacol. 31: 800 (1979) hingewiesen. Demgegenüber wurde durch Zugabe von 2% Maisstärke die Adhäsion der Wirkstoffpartikel verstärkt und die an Sucrose haftende Wirkstoffmenge erhöht, während durch Zugabe von 2% Talk die Adhäsionskräfte zwischen den Teilchen allgemein erhöht wurden. Ähnliche Effekte wurden auch von N.M. Kassem [Doktorarbeit DX187842, Universität London, 1990] gefunden und ebenfalls durch die elektrostatischen Eigenschaften der Bestandteile erklärt.

In WO-A-87/05213 wurde andererseits vorgeschlagen, zur Herstellung von Inhalationspulvern Träger, bestehend aus Mikrokörnchen eines Konglomerats eines oder mehrerer fester wasserlöslicher Verdünnungsmittel, wie Lactose, Xylitol, Mannitol, Arabinose oder Dextran, mit einem Schmiermittel, wie Magnesiumstearat, Natriumbenzoat, kolloidalem Silika, hydriertem Öl oder Fettsubstanzen, zu verwenden. Die Mikrokörnchen haben vorzugsweise eine Korngrösse von 30-150 µm und werden dadurch hergestellt, dass das Schmiermittel einer wässrigen Lösung eines Teils des festen Verdünnungsmittels zugesetzt, das restliche Verdünnungsmittel zusammen mit dieser Mischung granuliert und das erhaltene Granulat gesiebt wird. Die Verwendung solcher Träger soll u.a. verbesserte Fliesseigenschaften und verbesserte selbstschmierende Eigenschaften ermöglichen.

Es hat sich jedoch gezeigt, dass Pulvermischungen, insbesondere interaktive Pulvermischungen, sensititv gegenüber der Feuchtigkeit in der Umgebungsluft sind. Sie sind daher für den Einsatz in einem Multidosis-Trockenpulverinhalator, der ein Pulverreservoir enthält, nur bedingt geeignet, da dieser normalerweise keine dichte Verpackung im Sinne einer hermetischen Absperrung von Wasserdampf darstellt. Dies äussert sich meist in einem dramatischen Abfall des inhalierbaren Anteils der abgegebenen Dosis, die in vitro als FPD oder FPF bestimmt wird. Der Abfall beruht auf einer stärkeren Adhäsion der mikroniserten Wirkstoffpartikel an den Trägerpartikeln, da ab einer relativen Luftfeuchtigkeit von ca. 60 % durch Wasserdampfkondensation sogenannte Flüssigkeitsbrücken in den Zwischenräumen entstehen, die zu einer stärkeren Bindungsenergie beitragen. Visuell sichtbares Zeichen für diesen Vorgang sind Krusten-oder Klumpenbildung, die aber nicht in jedem Fall notwendigerweise beobachtet werden müssen. Der Vorgang ist irreversibel, da beim Auftrocknen der Flüssigkeitsbrücken sogenannte feste Brücken entstehen. Unter anderem ist die Wasseraufnahmetendenz bzw. das Wassersorptionsvermögen der beteiligten Stoffe mitentscheidend für das Ausmass der Verschlechterung der Pulvereigenschaften bei hoher Luftfeuchtelagerung.

Der Erfindung liegt daher die Aufgabe zugrunde, die Sensitivität von Pulvermischungen gegenüber Feuchtigkeit zu verringern. Die Aufgabe wird erfindungsgemäss gelöst durch Verwendung von Magnesiumstearat. Es hat sich nämlich überraschenderweise gezeigt, dass Magnesiumstearat in der Lage ist, den Einfluss von eindringender Feuchtigkeit auf die FPD und die FPF während der Lagerung des Inhalationspulvers zu minimieren, d.h. eine durch Feuchtigkeit bedingte Verschlechterung der FPD und der FPF zu verhindern oder zumindest erheblich zu verlangsamen, und die Trockenpulver-Formulierung zu stabilisieren. Die ursprüngliche Qualität der Arzneizubereitung bleibt also auch bei Lagerung unter extremen Bedingungen der Temperatur und Feuchte wesentlich besser als bei herkömmlichen Präparaten. Die Verbesserung äussert sich meist auch dadurch, dass der Einfluss von Feuchtigkeit auf das Massenmittel des aerodynamischen Teilchendurchmessers (Mass Median Aerodynamic Diameter, nachfolgend auch als MMAD bezeichnet) und auf die Genauigkeit und Reproduzierbarkeit der abgegebenen Dosis verhindert oder stark verlangsamt werden kann. Diese Effekte sind speziell für feuchtigkeitsempfindliche Wirkstoffe besonders ausgeprägt, da eine eventuelle Hygroskopizität des Wirkstoffes die Wasseraufnahme und damit die Ausbildung der Flüssigkeitsbrücken begünstigt. Darüber hinaus führt die Verwendung von Magnesiumstearat in der Regel zu einer generellen Verbesserung der FPD und der FPF. Es ist denkbar, dass das Magnesiumstearat neben einem generellen Feuchtigkeitsschutz die Trägermaterialien und Wirkstoffe auch in der Weise stabilisiert, dass unerwünschte morphologische Phasenübergänge unterbunden oder verlangsamt werden.

Die Erfindung betrifft daher die Verwendung von Magnesiumstearat zur Verbesserung der Feuchtigkeitsbeständigkeit, d.h. zur Verringerung der Sensitivität gegenüber Luftfeuchtigkeit, von Trockenpulver-Formulierungen zur Inhalation. Die Verwendung von Magnesiumstearat bewirkt dementsprechend eine Verbesserung der Lagerstabilität und insbesondere eine Verringerung des Einflusses von eindringender Feuchtigkeit auf die FPF (und die FPD), was die Aufrechterhaltung einer hohen FPD und FPF auch unter vergleichsweise extremen Temperatur- und Feuchtigkeitsbedingungen gestattet.

Die erfindungsgemäss erhältlichen Trockenpulverformulierungen umfassen somit einen pharmazeutisch nicht wirksamen Träger in nicht inhalierbarer Teilchengrösse, einen fein verteilten pharmazeutischen Wirkstoff in inhalierbarer Teilchengrösse (d.h. mit einem mittleren Teilchendurchmesser von vorzugsweise höchstens 10 µm, insbesondere höchstens 5 µm) und - zur Verbesserung der Feuchtigkeitsbeständigkeit - Magnesiumstearat, und sie liegen vorzugsweise in Form von sogenannten interaktiven (oder geordneten oder adhäsiven) Mischungen vor. Gewünschtenfalls können die Trockenpulver-Formulierungen auch einen Anteil an Trägermaterial in inhalierbarer Teilchengrösse enthalten.

Der Ausdruck "interaktive Mischung" oder "geordnete Mischung" oder "adhäsive Mischung" ist dem Fachmann geläufig und umfasst im Rahmen der vorliegenden Erfindung Trockenpulver-Formulierungen, in denen der pharmakologisch inaktive Träger in einer Partikelgrösse vorliegt, die nicht inhalierbar oder überwiegend nicht inhalierbar ist, und in denen mikrofeine Wirkstoffpartikel durch Adhäsion an die Trägerpartikel gebunden (d.h. nicht im Träger z.B. in Form eines Granulates enthalten) sind.

Es wurde gefunden, dass sich Magnesiumstearat zur Verbesserung der Feuchtigkeitsbeständigkeit grundsätzlich beliebiger Trockenpulver-Formulierungen, unabhängig von der Art der Wirkstoffe und Trägermaterialien, eignet. Besonders ausgeprägt ist die Verbesserung jedoch im Falle von Trockenpulvern, deren Kombination von Wirkstoff und Träger - d.h. ohne Zusatz von Magnesiumstearat - eine hohe Sensitivität gegenüber dem Einfluss von Luftfeuchtigkeit aufweist und beispielsweise bei offener Lagerung bei 40°C und 75% relativer Luftfeuchtigkeit innert 10 Tagen eine Abnahme der FPF um mindestens 50% zeigt. Eine hohe Empfindlichkeit der FPF oder FPD auf Luftfeuchtigkeit wird häufig dann beobachtet, wenn der Wirkstoff in Form eines Salzes oder Esters vorliegt und/oder vergleichsweise hygroskopisch oder hydrophil ist.

Hygroskopisch ist in diesem Sinne ein Wirkstoff dann, wenn er bei einem Wasserdampfdruck in der Trocknungsluft > 0, d.h. in Kontakt mit Luft mit einem Feuchtigkeitsgehalt > 0% relativer Feuchte, nie vollständig austrocknet, sondern immer eine bestimmte Menge absorptiv gebundenes Wasser enthält [H. Sucker, P. Fuchs und P. Speiser: Pharmazeutische Technologie, Georg Thieme Verlag, Stuttgart, New York, 2. Auflage 1991, Seite 85]. Besonders vorteilhaft ist die erfindungsgemässe Verwendung von Magensiumstearat, wenn der Wirkstoff vergleichsweise hygroskopisch ist und beispielsweise bei Lagerung in Trocknungsluft mit einer relativen Feuchte von 50% mindestens etwa 0,5 Gew.-% adorptiv gebundenes Wasser aufnimmt bzw. behält.

Hydrophil ist ein Wirkstoffpulver dann, wenn es von Wasser leicht benetzt werden kann, wobei im Rahmen der vorliegenden Erfindung unter hydrophilen Wirkstoffpulvern insbesondere solche zu verstehen sind, die beispielsweise einen Benetzungswinkel von weniger als 90° aufweisen [Martin, Swarbrick und Cammarata: Physikalische Pharmazie, Wissenschaftliche Verlagsgesellschaft mbH, Stuttgart, 3. Auflage 1987, Seite 534]. Besonders vorteilhaft ist die erfindungsgemässe Verwendung von Magnesiumstearat im Falle von Wirkstoffpulvern, die einen Benetzungswinkel von weniger als 70° aufweisen.

Die Verwendung von Magnesiumstearat zur Verbesserung der Feuchtigkeitsbeständigkeit von Trockenpulver-Formulierungen ist somit besonders bevorzugt im Falle von Trockenpulver-Formulierungen, die einen pharmazeutischen Wirkstoff enthalten, der in Form eines Salzes oder Esters vorliegt und/oder bei Lagerung in Trocknungsluft mit einer relativen Feuchte von 50% mindestens etwa 0,5 Gew.-% adorptiv gebundenes Wasser aufnimmt bzw. behält und/oder einen Benetzungswinkel von weniger als 90°, insbesondere weniger als 70°, aufweist.

Die erfindungsgemässe Verwendung von Magnesiumstearat ist ferner vor allem für den Einsatz in Multidosis-Trockenpulverinhalatoren vorteilhaft, die ein Pulverreservoir enthalten, aus dem die einzelnen Dosen über einen Dosiermechanismus entnommen werden. Die Verwendung von Magnesiumstearat ist aber ebenfalls zur Verbesserung der Feuchtigkeitsbeständigkeit von vordosierten Einheiten geeignet, die beispielsweise in Form von Kapseln vorliegen können.

Der in den erfindungsgemäss erhältlichen Formulierungen vorhandene Wirkstoff kann grundsätzlich ein beliebiger, in Trockenpulvern inhalativ verabreichbarer pharmazeutischer Wirkstoff sein. Damit der Wirkstoff inhalierbar ist, d.h. in die Lunge gelangen kann, muss er in Partikeln mit einem mittleren Teilchendurchmesser (gemessen als MMAD) von höchstens etwa 10 µm, beispielsweise etwa 1 bis 10 µm und vorzugsweise etwa 1 bis 6 µm, vorliegen. Solche mikrofeinen Partikel können in bekannter oder an sich bekannter Weise, beispielsweise durch Mikronisierung, kontrollierte Ausfällung aus geeigneten Solventien (z.B. auch aus superkritischem Kohlendioxid) oder durch Sprühtrocknung, erhalten werden, wenn die Verfahrensbedingungen geeignet gewählt, kontrolliert und ausgeführt werden.

Die erfindungsgemäss erhältlichen Formulierungen können als Wirkstoff vorzugsweise ein Betamimetikum, wie Levalbuterol, Terbutalin, Reproterol, Salbutamol, Salmeterol, Formoterol, Fenoterol, Clenbuterol, Bambuterol, Tulobuterol, Broxaterol, Epinephrin, Isoprenalin oder Hexoprenalin, ein Anticholinergikum, wie Tiotropium, Ipratropium, Oxitropium oder Glycopyrronium, ein Corticosteroid, wie Butixocart, Rofleponid, Budesonid, Ciclosenid, Mometason, Fluticason, Beclomethason, Loteprednol oder Triamcinolon, einen Leukotrienantagonisten, wie Andolast, Iralukast, Pranlukast, Imitrodast, Seratrodast, Zileuton, Zafirlukast oder Montelukast, einen Phosphodiesterase-Inhibitor, wie Filaminast oder Piclamilast, einen PAF-Inhibitor, wie Apafant, Forapafant oder Israpafant, einen Kaliumkanalöffner, wie Amilorid oder Furosemid, ein Schmerzmittel, wie Morphin, Fentanyl, Pentazozin, Buprenorphin, Pethidin, Tilidin, Methadon oder Heroin, ein Potenzmittel, wie Sildenafil, Alprostadil oder Phentolamin, ein Peptid oder Protein, wie Insulin, Erythropoietin, Gonadotropin oder Vasopressin, oder ein pharmazeutisch annehmbares Derivat oder Salz dieser Verbindungen enthalten. Im Falle von chiralen Wirkstoffen kann dieser in Form eines optischen Isomers, eines Diastereoisomerengemisches oder Racemates vorliegen. Gewünschtenfalls können die erfindungsgemässen Formulierungen zwei oder mehrere pharmazeutische Wirkstoffe enthalten.

Da die Feuchtigkeitsempfindlichkeit vor allem im Falle von Wirkstoffen, die als Salz oder Ester vorliegen, häufig ein grosses Problem darstellt, ist der Einsatz von Magnesiumstearat insbesondere bei Trockenpulver-Formulierungen vorteilhaft, die mindestens einen pharmazeutischen Wirkstoff in Form eines pharmazeutisch annehmbaren Salzes, beispielsweise ein Chlorid, Bromid, Iodid, Nitrat, Carbonat, Sulfat, Methylsulfat, Phosphat, Acetat, Benzoat, Benzolsulfonat, Fumarat, Malonat, Tartrat, Succinat, Citrat, Lactat, Gluconat, Glutamat, Edetat, Mesylat, Pamoat, Pantothenat oder Hydroxynaphthoat, oder einen pharmazeutischen Wirkstoff in Form eines pharmazeutisch annehmbaren Esters, beispielsweise ein Acetat, Propionat, Phosphat, Succinat oder Etabonat, enthalten.

Besonders bevorzugt ist die Verwendung von Magnesiumstearat in Trockenpulver-Formulierungen, die ein Betamimetikum und/oder ein Anticholinergikum und/oder ein Corticosteroid enthalten, und insbesondere in Trockenpulver-Formulierungen, die ein Betamimetikum und/oder ein Anticholinergikum und/oder ein Corticosteroid in Form eines pharmazeutisch annehmbaren Salzes oder Esters enthalten, beispielsweise ein Betamimetikum in Form eines Salzes, wie z.B. Levalbuterolsulfat, Formoterolfumarat, Formoteroltartrat, Salbutamolsulfat oder Salmeterolxinafoat (Salmeterol-1-hydroxy-2-naphthoat), oder ein Anticholinergikum in Form eines Salzes, wie z.B. Oxitropiumbromid, Glycopyrrolat (Glycopyrroniumbromid), Ipratropiumbromid oder Tiotropiumbromid, oder ein Corticosteroid in Form eines Esters, wie z.B. Beclomethason-dipropiponat, Fluticason-propionat, Triamcinolon-16,21-diacetat, Triamcinolonacetonid-21-acetat, Triamcinolonacetonid-21-dinatriumphosphat, Triamcinolonacetonid-21-hemisuccinat, Mometasonfuroat oder Loteprednol-etabonat, oder eine Kombination davon, wie beispielsweise Ipratropiumbromid in Kombination mit Salbutamolsulfat.

Gemäss einem weiteren bevorzugten Aspekt können die erfindungsgemäss erhältlichen Formulierungen insbesondere auch ein Corticosteroid, wie Ciclosenid, Rofleponid, Fluticason-propionat, Mometasonfuroat oder Loteprednol-etabonat, in Kombination mit einem Betamimetikum, wie Formoterolfumarat, Formoteroltartrat, Levalbuterolsulfat oder Salmeterolxinafoat, enthalten.

Die Wirkstoffmenge in den erfindungsgemäss erhältlichen Formulierungen kann in breiten Bereichen variieren und ist in hohem Masse vom jeweiligen Wirkstoff und bis zu einem gewissen Grad auch vom verwendeten Pulverinhalator abhängig. Typischerweise kann die Wirkstoffkonzentration etwa 0,1 bis 10 Gew.-%, insbesondere etwa 0,1 bis 5 Gew.-%, bezogen auf die Gesamtformulierung, betragen. Gelegentlich können auch höhere oder tiefere Konzentrationen zweckmässig sein, wobei aber Wirkstoffkonzentrationen unter 0,001 Gew.-% oder unter 0,01 Gew.-% selten vorkommen.

Zur exakten volumetrischen Dosierung der meisten Wirkstoffe bzw. Formulierungen ist eine Verdünnung des Wirkstoffes mit einem pharmazeutisch inaktiven Hilfsstoff erforderlich, um eine den Anforderungen an die Dosiergenauigkeit genügende, dosierbare Einheitsmenge zu erhalten. Hierzu werden die mikrofeinen, inhalierbaren Wirkstoffpartikel mit pharmakologisch inaktiven Substanzen (Trägern) gemischt. Die Verdünnung wird dabei so gewählt, dass die vom Pulverinhalator ausgebrachte Menge genau die gewünschte Dosis enthält. Der pharmakologisch inaktive Hilfsstoff dient vorzugsweise nicht nur zum Verdünnen, sondern auch zum Einstellen einer möglichst guten Fliessfähigkeit der Pulvermischung, und er ist im Falle der bevorzugt verwendeten sogenannten interaktiven oder geordneten Mischungen die Trägersubstanz, an die die mikrofeinen Wirkstoffpartikel durch Adhäsion gebunden werden, um so eine geeignete Mischgüte, d.h. Homogenität der Mischung, zu erreichen und aufrecht zu erhalten.

Der Träger liegt in der erfindungsgemäss erhältlichen Formulierung vorzugsweise in einer Partikelgrösse vor, die nicht inhalierbar ist. Die Trägerteilchen sollten aber andererseits nicht zu gross sein, da sich dies nachteilig auf die FPF auswirken kann. Die optimale Partikelgrösse des eingesetzten Trägers richtet sich dabei in der Regel nach den Anforderungen und Gegebenheiten des Pulverinhalators, der für die Applikation der Formulierung vorgesehen ist. Im Rahmen der vorliegenden Erfindung können Träger mit üblichen Teilchengrössen verwendet werden, und optimale Teilchengrössen können vom Fachmann von Fall zu Fall leicht ermittelt werden. Im allgemeinen kann jedoch der mittlere Teilchendurchmesser (MMAD) der Trägerpartikel etwa 10 bis 500 µm und vorzugsweise etwa 50 bis 200 µm betragen.

Die Haftung der Wirkstoffpartikel an den Trägerteilchen sollte ausreichend sein, dass während den Verarbeitungs-, Transport-, Lager- und Dosiervorgängen keine Entmischung stattfindet, aber andererseits nicht so hoch, dass eine möglichst quantitative Ablösung der Wirkstoffteilchen während dem Dispergieren im Inhalator, ausgelöst durch den Atemfluss des Patienten, nicht mehr gewährleistet ist. Die Effektivität der Freisetzung der Wirkstoffpartikel ist neben den physikalisch-chemischen Eigenschaften des Wirkstoffes und den aerodynamischen Eigenschaften des Pulverinhalators vor allem von den Eigenschaften des Trägers, insbesondere der Art des Trägers und dessen Oberflächenstruktur, mittlerer Partikelgrösse und Partikelgrössenverteilung, abhängig.

Im Rahmen der vorliegenden Erfindung sind grundsätzlich alle üblicherweise in Trockenpulver-Formulierungen verwendeten Trägermaterialien geeignet, beispielsweise Mono- oder Disaccharide, wie Glucose, Lactose, Lactose-Monohydrat, Saccharose oder Trehalose, Zuckeralkohole, wie Mannitol oder Xylitol, Polymilchsäure oder Cyclodextrin, wobei Glucose, Trehalose und insbesondere Lactose-Monohydrat im allgemeinen bevorzugt sind. Gewünschtenfalls können die Formulierungen auch zwei oder mehrere Trägermaterialien enthalten. Gewünschtenfalls kann die Formulierung neben nicht inhalierbaren Trägerpartikeln auch einen Anteil an inhalierbaren Trägerpartikeln enthalten; zum Beispiel kann sie neben gröberen Lactose-Monohydrat-Trägerpartikeln einen Anteil von z.B. 0,1 bis 10 Gew.-% an mikronisiertem Lactose-Monohydrat enthalten, das beispielsweise für mindestens 50% der Partikel einen Teilchendurchmesser von höchstens 10 µm, vorzugsweise höchstens 5 µm, aufweisen kann.

Der Anteil an Trägermaterial in den erfindungsgemäss erhältlichen Formulierungen kann je nach der für den jeweiligen Wirkstoff nötigen oder wünschbaren Verdünnung und der zur Verbesserung der Feuchtigkeitsbeständigkeit verwendeten Menge an Magnesiumstearat in einem breiten Bereich variieren. Der Anteil an Trägermaterial in der Gesamtformulierung kann beispielsweise etwa 80 bis 99,9 Gew.-% betragen, wobei aber je nach Wirkstoff auch höhere oder niedrigere Anteile vorteilhaft sein können.

Die Konzentration an Magnesiumstearat kann ebenfalls innerhalb relativ breiter Grenzen variieren und beispielsweise etwa 0,001 bis 10 Gew.-%, insbesondere etwa 0,01 bis 5 Gew.-%, bezogen auf die Gesamtformulierung, betragen, wobei eine Konzentration von etwa 0,1 bis 2 Gew.-% in der Regel bevorzugt ist. Im Hinblick auf toxikologische Unbedenklichkeit wird die Magnesiumstearat-Konzentration jedoch zumeist nicht über etwa 1 Gew.-% liegen, aber andererseits meist mindestens etwa 0,25 Gew.-% betragen, um eine hohe Wirksamkeit zu gewährleisten, wobei sich ein Konzentrationsbereich von etwa 0,4 bis 0,8 Gew.-%, vorzugsweise etwa 0,5 bis 0,75 Gew.-%, für die meisten Fälle besonders bewährt hat. Das Magnesiumstearat wird vorzugsweise als staubförmiges Material eingesetzt; die Teilchengrösse ist nicht besonders kritisch.

Die erfindungsgemäss erhältlichen Formulierungen können gewünschtenfalls weitere Komponenten enthalten. Vorzugsweise bestehen sie jedoch aus einem oder mehreren pharmazeutisch nicht wirksamen Trägern, einem oder mehreren pharmazeutischen Wirkstoffen und Magnesiumstearat.

Die Trockenpulver-Formulierungen können erfindungsgemäss dadurch hergestellt werden, dass man einen pharmazeutisch nicht wirksamen Träger in nicht inhalierbarer Teilchengrösse (der gewünschtenfalls einen Anteil in inhalierbarer Teilchengrösse enthalten kann), einen fein verteilten pharmazeutischen Wirkstoff in inhalierbarer Teilchengrösse, beispielsweise mit einem mittleren Teilchendurchmesser von höchstens 10 µm (vorzugsweise höchstens 5 µm), und Magnesiumstearat miteinander vermischt. Die Bestandteile können grundsätzlich in beliebiger Reihenfolge miteinander gemischt werden, wobei aber die Vermischung zweckmässigerweise so erfolgen sollte, dass die Partikel der Bestandteile - abgesehen von der Adhäsion an die Trägerpartikel - im wesentlichen als solche erhalten bleiben, d.h. nicht z.B. durch eine Granulierung und dergleichen zerstört werden. Gemäss einer bevorzugten Variante kann jedoch zuerst eine Vormischung von Magnesiumstearat mit dem Träger hergestellt und dann die Wirkstoffpartikel zugemischt werden. Gemäss einer weiteren bevorzugten Variante kann zuerst eine Vormischung des Wirkstoffes mit dem Träger hergestellt und dann das Magnesiumstearat zugemischt werden. Das Mischen kann in an sich bekannter Weise, beispielsweise in einem Taumelmischer, erfolgen. Vorzugsweise kann in diesen Verfahren jeweils staubförmiges Magnesiumstearat mit einer mittleren Teilchengrösse und etwa 1 bis 100 µm, insbesondere etwa 5 bis 20 µm, zugesetzt werden.

Die beschriebenen Trockenpulver-Formulierungen können in allen üblichen Trockenpulverinhalatoren angewendet werden. Besonders vorteilhaft sind sie zur Anwendung in Multidosis-Trockenpulverinhalatoren, die ein Pulverreservoir enthalten, insbesondere in Multidosis-Pulverinhalatoren wie in WO-A-97/20589 beschrieben.

Die Erfindung betrifft ebenfalls Trockenpulver-Formulierungen zur Inhalation mit verbesserter Feuchtigkeitsbeständigkeit, umfassend einen pharmazeutisch nicht wirksamen Träger in nicht inhalierbarer Teilchengrösse, einen fein verteilten pharmazeutischen Wirkstoff in Form eines pharmazeutisch annehmbaren Salzes oder Esters in inhalierbarer Teilchengrösse (vorzugsweise mit einem mittleren Teilchendurchmesser von höchstens 10 µm, insbesondere höchstens 5 µm) und 0,25 bis 1 Gew.-%, bezogen auf die Gesamtformulierung, an Magnesiumstearat. Bevorzugt sind Trockenpulver-Formulierungen, die in Form von interaktiven Mischungen vorliegen. Bevorzugte Wirkstoffsalze und -ester, Trägermaterialien, Mengenbereiche, Methoden und dergleichen ergeben sich aus der vorangehenden Beschreibung.

Die Erfindung wird durch die folgenden Beispiele weiter veranschaulicht. In den Beispielen bezeichnet r.F. die relative Luftfeuchtigkeit; die Bezeichnung n.b. gibt an, dass der betreffende Wert nicht bestimmt wurde. Die Tests erfolgten jeweils mit einem Trockenpulverinhalator vom Typ SkyePharma mDPI (SkyePharma AG, Schweiz) gemäss WO-A-97/20589. Die Bestimmung der FPD und der FPF erfolgte - sofern nicht anders angegeben - jeweils mit einem Twin Impinger. Siebungen erfolgten - sofern nicht anders angegeben - jeweils mit einem Sieb mit einer Lochweite von 180 µm. Zur Ermittlung der Feuchtigkeitsempfindlichkeit wurden die Trockenpulver, ausser im Beispiel 7, jeweils offen ohne Feuchtigkeitsschutz gelagert.

Die vorliegende Erfindung umfasst auch die in den folgenden Absätzen 1 bis 30 offenbarten Ausführungsformen:
1. Verwendung von Magnesiumstearat in Trockenpulver-Formulierungen zur Inhalation zwecks Verbesserung der Feuchtigkeitsbeständigkeit.
2. Verwendung von Magnesiumstearat in Trockenpulver-Formulierungen zur Inhalation zwecks Verringerung des Einflusses von eindringender Feuchtigkeit auf den Feinpartikelanteil (FPF) .
3. Verwendung gemäss dem vorstehenden Absatz 1 oder 2, wobei die Trockenpulver-Formulierung einen pharmazeutisch nicht wirksamen Träger in nicht inhalierbarer Teilchengrösse, einen fein verteilten pharmazeutischen Wirkstoff in inhalierbarer Teilchengrösse und Magnesiumstearat umfasst und in Form einer interaktiven Mischung vorliegt.
4. Verwendung gemäss einem der vorstehenden Absätze 1 bis 3, wobei das Magnesiumstearat in einer Konzentration von 0,1 bis 2 Gew.-%, bezogen auf die Gesamtformulierung, eingesetzt wird.
5. Verwendung gemäss einem der vorstehenden Absätze 1 bis 4, wobei das Magnesiumstearat in einer Konzentration von 0,25 bis 1 Gew.-%, vorzugsweise 0,4 bis 0,8 Gew.-%, bezogen auf die Gesamtformulierung, eingesetzt wird.
6. Verwendung gemäss einem der vorstehenden Absätze 1 bis 5, wobei die Kombination von Wirkstoff und Träger eine hohe Sensitivität gegenüber dem Einfluss Luftfeuchtigkeit aufweist.
7. Verwendung gemäss einem der vorstehenden Absätze 1 bis 6, wobei die Trockenpulver-Formulierung als pharmazeutischen Wirkstoff ein Betamimetikum, ein Anticholinergikum, ein Corticosteroid, einen Leukorienantagonisten, einen Phosphodiesterase-Inhibitor, einen PAF-Inhibitor, einen Kaliumkanalöffner, ein Schmerzmittel, ein Potenzmittel, ein Peptid oder ein Protein, vorzugsweise ein Betamimetikum und/oder ein Anticholinergikum und/oder ein Corticosteroid, enthält.
8. Verwendung gemäss einem der vorstehenden Absätze 1 bis 7, wobei die Trockenpulver-Formulierung als pharmazeutischen Wirkstoff ein Betamimetikum aus der Gruppe, umfassend Levalbuterol, Terbutalin, Reproterol, Salbutamol, Salmeterol, Formoterol, Fenoterol, Clenbuterol, Bambuterol, Tulobuterol, Broxaterol, Epinephrin, Isoprenalin und Hexoprenalin, ein Anticholinergikum aus der Gruppe, umfassend Tiotropium, Ipratropium, Oxitropium und Glycopyrronium, ein Corticosteroid aus der Gruppe, umfassend Butixocart, Rofleponid, Budesonid, Ciclosenid, Mometason, Fluticason, Beclomethason, Loteprednol und Triamcinolon, einen Leukotrienantagonisten aus der Gruppe, umfassend Andolast, Iralukast, Pranlukast, Imitrodast, Seratrodast, Zileuton, Zafirlukast und Montelukast, einen Phosphodiesterase-Inhibitor aus der Gruppe, umfassend Filaminast und Piclamilast, einen PAF-Inhibitor aus der Gruppe, umfassend Apafant, Forapafant und Israpafant, einen Kaliumkanalöffner aus der Gruppe, umfassend Amilorid und Furosemid, ein Schmerzmittel aus der Gruppe, umfassend Morphin, Fentanyl, Pentazozin, Buprenorphin, Pethidin, Tilidin, Methadon und Heroin, ein Potenzmittel aus der Gruppe, umfassend Sildenafil, Alprostadil und Phentolamin, ein Peptid oder Protein aus der Gruppe, umfassend Insulin, Erythropoietin, Gonadotropin und Vasopressin, oder ein pharmazeutisch annehmbares Derivat oder Salz einer dieser Verbindungen enthält.
9. Verwendung gemäss einem der vorstehenden Absätze 1 bis 8, wobei die Trockenpulver-Formulierung als pharmazeutischen Wirkstoff ein Betamimetikum aus der Gruppe, umfassend Salbutamol, Salmeterol, Formoterol, Fenoterol, Clenbuterol, Bambuterol, Tulobuterol, Broxaterol, Epinephrin, Isoprenalin und Hexoprenalin, ein Anticholinergikum aus der Gruppe, umfassend Tiotropium, Ipratropium, Oxitropium und Glycopyrronium, ein Corticosteroid aus der Gruppe, umfassend Budesonid, Ciclosenid, Mometason, Fluticason, Beclomethason, Loteprednol und Triamcinolon, einen Leukotrienantagonisten aus der Gruppe, umfassend Zileuton, Zafirlukast und Montelukast, einen Kaliumkanalöffner aus der Gruppe, umfassend Amilorid und Furosemid, ein Schmerzmittel aus der Gruppe, umfassend Morphin, Fentanyl, Pentazozin, Buprenorphin, Pethidin, Tilidin, Methadon und Heroin, ein Potenzmittel aus der Gruppe, umfassend Sildenafil, Alprostadil und Phentolamin, ein Peptid oder Protein aus der Gruppe, umfassend Insulin, Erythropoietin, Gonadotropin und Vasopressin, oder ein pharmazeutisch annehmbares Derivat oder Salz einer dieser Verbindungen enthält.
10. Verwendung gemäss einem der vorstehenden Absätze 1 bis 9, wobei die Trockenpulver-Formulierung einen pharmazeutischen Wirkstoff enthält, der in Form eines pharmazeutisch annehmbaren Salzes oder Esters vorliegt.
11. Verwendung gemäss einem der vorstehenden Absätze 1 bis 10, wobei die Trockenpulver-Formulierung als pharmazeutischen Wirkstoff ein Betamimetikum und/oder ein Anticholinergikum und/oder ein Corticosteroid enthält, das in Form eines pharmazeutisch annehmbaren Salzes oder Esters vorliegt.
12. Verwendung gemäss einem der vorstehenden Absätze 1 bis 11, wobei die Trockenpulver-Formulierung als pharmazeutischen Wirkstoff ein Betamimetikum aus der Gruppe, umfassend Levalbuterolsulfat, Formoterolfumarat, Formoteroltartrat, Salbutamolsulfat und Salmeterolxinafoat, und/oder ein Anticholinergikum aus der Gruppe, umfassend Oxitropiumbromid, Glycopyrrolat, Ipratropiumbromid und Tiotropiumbromid, enthält.
13. Verwendung gemäss einem der vorstehenden Absätze 1 bis 12, wobei die Trockenpulver-Formulierung als pharmazeutischen Wirkstoff Formoterol oder ein pharmazeutisch annehmbares Salz davon enthält.
14. Verwendung gemäss einem der vorstehenden Absätze 1 bis 13, wobei die Trockenpulver-Formulierung als pharmazeutischen Wirkstoff ein Corticosteroid aus der Gruppe, umfassend Beclomethason-dipropiponat, Fluticason-propionat, Triamcinolon-16,21-diacetat, Triamcinolonacetonid-21-acetat, Triamcinolonacetonid-21-dinatriumphosphat, Triamcinolonacetonid-21-hemisuccinat, Mometasonfuroat und Loteprednol-etabonat, enthält.
15. Verwendung gemäss einem der vorstehenden Absätze 1 bis 14, wobei die Trockenpulver-Formulierung als pharmazeutischen Wirkstoff ein Corticosteroid in Kombination mit einem Betamimetikum, vorzugsweise ein Corticosteroid aus der Gruppe, umfassend Ciclosenid, Rofleponid, Fluticason-propionat, Mometasonfuroat und Loteprednol-etabonat, in Kombination mit einem Betamimetikum aus der Gruppe, umfassend Formoterolfumarat, Formoteroltartrat, Levalbuterolsulfat und Salmeterolxinafoat, enthält.
16. Verwendung gemäss einem der vorstehenden Absätze 1 bis 15, wobei die Trockenpulver-Formulierung als Träger ein Mono- oder Disaccharid, einen Zuckeralkohol, Polymilchsäure oder Cyclodetrin enthält.
17. Verwendung gemäss einem der vorstehenden Absätze 1 bis 16, wobei die Trockenpulver-Formulierung als Träger Glucose, Lactose-Monohydrat oder Trehalose enthält.
18. Verwendung gemäss einem der vorstehenden Absätze 1 bis 17, wobei man einen pharmazeutisch nicht wirksamen Träger in nicht inhalierbarer Teilchengrösse, einen fein verteilten pharmazeutischen Wirkstoff in inhalierbarer Teilchengrösse und Magnesiumstearat miteinander vermischt.
19. Verwendung gemäss dem vorstehenden Absatz 18, wobei eine Vormischung von Magnesiumstearat mit dem Träger hergestellt und dann der Wirkstoff zugemischt wird.
20. Verwendung gemäss dem vorstehenden Absatz 18, wobei eine Vormischung des Wirkstoffes mit dem Träger hergestellt und dann das Magnesiumstearat zugemischt wird.
21. Verwendung gemäss einem der vorstehenden Absätze 1 bis 20, zum Einsatz in einem Trockenpulverinhalator, vorzugsweise einem Multidosis-Trockenpulverinhalator, der ein Pulverreservoir enthält.
22. Trockenpulver-Formulierung zur Inhalation mit verbesserter Feuchtigkeitsbeständigkeit, umfassend einen pharmazeutisch nicht wirksamen Träger in nicht inhalierbarer Teilchengrösse, einen fein verteilten pharmazeutischen Wirkstoff in Form eines pharmazeutisch annehmbaren Salzes oder Esters in inhalierbarer Teilchengrösse und 0,25 bis 1 Gew.-%, bezogen auf die Gesamtformulierung, an Magnesiumstearat.
23. Trockenpulver-Formulierung gemäss dem vorstehenden Absatz 22, wobei das Magnesiumstearat in einer Menge von 0,4 bis 0,8 Gew.-%, vorzugsweise 0,5 bis 0,75 Gew.-%, bezogen auf die Gesamtformulierung, vorliegt.
24. Trockenpulver-Formulierung gemäss einem der vorstehenden Absätze 22 oder 23, wobei sie als pharmazeutischen Wirkstoff ein Betamimetikum, ein Anticholinergikum, ein Corticosteroid, einen Leukorienantagonisten, einen Phosphodiesterase-Inhibitor, einen PAF-Inhibitor, einen Kaliumkanalöffner, ein Schmerzmittel, ein Potenzmittel, ein Peptid oder ein Protein, vorzugsweise ein Betamimetikum und/oder ein Anticholinergikum und/oder ein Corticosteroid, enthält.
25. Trockenpulver-Formulierung gemäss einem der vorstehenden Absätze 22 bis 24, wobei sie als pharmazeutischen Wirkstoff ein Betamimetikum aus der Gruppe, umfassend Levalbuterolsulfat, Formoterolfumarat, Formoteroltartrat, Salbutamolsulfat und Salmeterolxinafoat, und/oder ein Anticholinergikum aus der Gruppe, umfassend Oxitropiumbromid, Glycopyrrolat, Ipratropiumbromid und Tiotropiumbromid, enthält.
26. Trockenpulver-Formulierung gemäss einem der vorstehenden Absätze 22 bis 25, wobei sie als pharmazeutischen Wirkstoff Formoterol in Form eines pharmazeutisch annehmbaren Salzes enthält.
27. Trockenpulver-Formulierung gemäss einem der vorstehenden Absätze 22 bis 26, wobei sie als pharmazeutischen Wirkstoff ein Corticosteroid aus der Gruppe, umfassend Beclomethason-dipropiponat, Fluticason-propionat, Triamcinolon-16,21-diacetat, Triamcinolonacetonid-21-acetat, Triamcinolonacetonid-21-dinatriumphosphat, Triamcinolonacetonid-21-hemisuccinat, Mometasonfuroat und Loteprednol-etabonat enthält.
28. Trockenpulver-Formulierung gemäss einem der vorstehenden Absätze 22 bis 27, wobei sie als pharmazeutischen Wirkstoff ein Corticosteroid in Kombination mit einem Betamimetikum, vorzugsweise ein Corticosteroid aus der Gruppe, umfassend Ciclosenid, Rofleponid, Fluticason-propionat, Mometasonfuroat und Loteprednol-etabonat, in Kombination mit einem Betamimetikum aus der Gruppe, umfassend Formoterolfumarat, Formoteroltartrat, Levalbuterolsulfat und Salmeterolxinafoat, enthält.
29. Trockenpulver-Formulierung gemäss einem der vorstehenden Absätze 22 bis 28, wobei sie als Träger Mono-oder Disaccharid, einen Zuckeralkohol, Polymilchsäure oder Cyclodetrin enthält.
30. Trockenpulver-Formulierung gemäss einem der vorstehenden Absätze 22 bis 29, wobei sie als Träger Glucose, Lactose-Monohydrat oder Trehalose enthält.

### Beispiel 1

198,46 g Lactose-Monohydrat mit einer definierten Korngrösse < 200 µm für 100%, < 125 µm für 50% und < 75 µm für 10% der Partikel(Siebanalyse) werden gesiebt und mit 1 g gesiebtem Magnesiumstearat mit einem Taumelmischer gemischt. Im Anschluss werden 0,54 g Formoterolfumarat-Dihydrat und die Vormischung gesiebt und gemischt. Die so erhaltene Mischung wird in einen geeigneten dosierenden Trockenpulverinhalator gefüllt. Für die genaue analytische Bestimmung der Partikelgrössenverteilung und speziell der FPD bzw. FPF wird eine adäquate Anzahl Dosierungen in einen im Europäischen Arzneibuch oder anderen nationalen Pharmakopöen beschriebenen Impaktor oder Impinger, z.B. den sogenannten Twin Impinger oder Multi Stage Liquid Impinger, nach ebenfalls dort beschriebenen Verfahren abgegeben und gesammelt. Die aufgefangenen und abgeschiedenen Wirkstoffpartikel werden in analytischen Standardverfahren zu Probelösungen aufgearbeitet und die in jeder Grössenklasse abgeschiedenen Wirkstoffmengen bestimmt. Zum Test der Stabilität gegen Feuchtigkeit werden Proben der Inhalationpulver offen bei 40°C / 75% r.F. oder einer anderen geeigneten Bedingung über einen Zeitraum von mehreren Tagen bis Wochen gelagert und anschliessend wie oben beschrieben im Pulverinhalator getestet.

Die mit dem hergestellten ternären Gemisch (Formulierung 1-A) und mit konventionellen Mischungen (Formulierungen 1-B und 1-C) in einem 5-Stufen Liquid Impinger gemäss Ph. Eur. erhaltenen Ergebnisse sowie die Zusammensetzungen der Mischungen (in Gew.-%) sind in Tabelle 1 aufgeführt. Im Vergleich zu den konventionellen interaktiven Mischungen zeigt die erfindungsgemässe ternäre Mischung mit Magnesiumstearat den Vorteil einer erhöhten FPD bzw. FPF und einer wesentlich verbesserten Stabilität der FPD bzw. FPF bei Lagerung bei 40°C / 75% r.F. Wie die Ergebnisse zu Formulierung 1-C zeigen, kann in konventionellen Formulierungen durch Zusatz von mikronisierter Lactose zwar eine anfängliche Erhöhung der FPD und FPF erreicht werden, nicht aber ein Schutz gegen den Einfluss erhöhter Temperatur und Feuchte. Dies ist ebenfalls aus den für die Formulierungen 1-A und 1-C unmittelbar nach Herstellung bzw. nach 7 oder 13 Tagen Lagerung des Trockenpulvers bei 40°C / 75% r.F. ermittelten MMAD-Werten ersichtlich: für Formulierung 1-A nach Herstellung 1,8 µm, nach 7 Tagen 1,9 µm und nach 13 Tagen 1,9 µm; für Formulierung 1-C nach Herstellung 2,2 µm, nach 7 Tagen 4,5 µm und nach 13 Tagen 5,5 µm. Im Gegensatz zur konventionellen Formulierung bleibt somit der MMAD bei der erfindungsgemässen Formulierung konstant, was die Ergebnisse der FPD- und FPF-Untersuchung bestätigt.

**Tabelle 1**

| Formulierung | 1-A | 1-B (Vergleich) | 1-C (Vergleich) |
|---|---|---|---|
| Lactose-Monohydrat | 99,23 % | 99,73 % | 97,24 % |
| Lactose-Monohydrat mikronisiert | 0,00 % | 0,00 % | 2,49 % |
| Magnesiumstearat | 0,50 % | 0,00 % | 0,00 % |
| Formoterolfumarat-Dihydrat mikronisiert | 0,27 % | 0,27 % | 0,27 % |
| FPD nach Herstellung [µg pro Hub] | 4,7 | 1,3 | 3,3 |
| FPD nach 3-4 Tagen bei 40°C/75% r.F. [µg pro Hub] | 4,5 | n.b. | 1,0 |
| FPF nach Herstellung [% gefundener Wirkstoff] | 42,5 | 13,7 | 35,9 |
| FPF nach 3-4 Tagen bei 40°C/75% r.F. [% gefundener Wirkstoff] | 37,3 | n.b. | 11,0 |

### Beispiel 2

97,23 g Lactose-Monohydrat mit einer definierten Korngrösse < 200 µm für 100%, < 125 µm für 50% und < 75 µm für 10% der Partikel (Siebanalyse) werden gesiebt und mit 2,5 g gesiebtem mikronisiertem Lactose-Monohydrat (50% der Partikel < 5 µm) in einem Taumelmischer gemischt. Im Anschluss werden 0,27 g Formoterolfumarat-Dihydrat und die Vormischung gesiebt und gemischt. Die so erhaltene Mischung wird mit 0,125 g gesiebtem Magnesiumstearat gemischt und in einen geeigneten dosierenden Trockenpulverinhalator gefüllt. Für die analytische Bestimmung der FPD bzw. FPF wird eine adäquate Anzahl Dosierungen in einen Twin Impinger oder Multi Stage Liquid Impinger abgegeben und gesammelt. Die aufgefangenen und abgeschiedenen Wirkstoffpartikel werden zu Probelösungen aufgearbeitet und die in jeder Grössenklasse abgeschiedenen Wirkstoffmengen bestimmt. Zum Test der Stabilität gegen Feuchtigkeit werden Proben der Inhalationpulver offen bei 40°C / 75% r.F. über einen Zeitraum von einigen Tagen gelagert und anschliessend wie oben beschrieben im Pulverinhalator getestet.

Die mit dem herstellten Gemisch (Formulierung 2) und mit einer konventionellen Mischung (Formulierung 1-C) in einem 5-Stufen Liquid Impinger gemäss Ph. Eur. erhaltenen Ergebnisse sowie die Zusammensetzungen der Mischungen (in Gew.-%) sind in Tabelle 2 aufgeführt. Im Vergleich zur konventionellen interaktiven Mischung zeigt die erfindungsgemässe Mischung mit Magnesiumstearat den Vorteil einer erhöhten FPD bzw. FPF und einer verbesserten Stabilität der FPD bzw. FPF bei Lagerung bei 40°C / 75% r.F.

**Tabelle 2**

| Formulierung | 2 | 1-C (Vergleich) |
|---|---|---|
| Lactose-Monohydrat | 96,75 % | 97,24 % |
| Lactose-Monohydrat mikronisiert | 2,48 % | 2,49 % |
| Magnesiumstearat | 0,50 % | 0,00 % |
| Formoterolfumarat-Dihydrat mikronisiert | 0,27 % | 0,27 |
| FPD nach Herstellung [µg pro Hub] | 5,3 | 3,3 |
| FPD nach 3-4 Tagen bei 40°C/75% r.F. [µg pro Hub] | n.b. | 1,0 |
| FPF nach Herstellung [% gefundener Wirkstoff] | 41,4 | 35,9 |
| FPF nach 3-4 Tagen bei 40°C/75% r.F. [% gefundener Wirkstoff] | n.b. | 11,0 |

### Beispiel 3

97 g Lactose-Monohydrat mit einer definierten Korngrösse < 110 µm für 90%, < 70 µm für 50% und < 40 µm für 10% der Partikel (Siebanalyse) werden gesiebt und mit 0,5 g gesiebtem Magnesiumstearat in einem Taumelmischer gemischt. Im Anschluss werden 2,5 g Salbutamolsulfat und die Vormischung gesiebt und gemischt. Die so erhaltene Mischung wird in einen geeigneten dosierenden Trockenpulverinhalator gefüllt. Für die analytische Bestimmung der FPD bzw. FPF wird eine adäquate Anzahl Dosierungen in einen Twin Impinger abgegeben und gesammelt. Die aufgefangenen und abgeschiedenen Wirkstoffpartikel werden zu Probelösungen aufgearbeitet und die in jeder Grössenklasse abgeschiedenen Wirkstoffmengen bestimmt. Zum Test der Stabilität gegen Feuchtigkeit werden Proben der Inhalationpulver offen bei 40 °C / 75 % r.F. über einen Zeitraum von 7 Tagen gelagert und anschliessend wie oben beschrieben im Pulverinhalator getestet.

Die mit dem herstellten ternären Gemisch (Formulierung 3-A) und mit einer konventionellen binären Mischung (Formulierung 3-B) in einem Twin Impinger gemäss Ph. Eur. erhaltenen Ergebnisse sowie die Zusammensetzungen der Mischungen (in Gew.-%) sind in Tabelle 3 aufgeführt. Die ternäre Mischung mit Magnesiumstearat erzielt eine höhere FPD bzw. FPF und ist wesentlich stabiler bei Lagerung bei 40°C / 75% r.F.

**Tabelle 3**

| Formulierung | 3-A | 3-B (Vergleich) |
|---|---|---|
| Lactose-Monohydrat | 97,00 % | 97,50 % |
| Magnesiumstearat | 0,50 % | 0,00 % |
| Salbutamolsulfat mikronisiert | 2,50 % | 2,50 % |
| FPD nach Herstellung [µg pro Hub] | 39,5 | 26,2 |
| FPD nach 7 Tagen bei 40°C/75% r.F. [µg pro Hub] | 27,8 | 11,3 |
| FPF nach Herstellung [% gefundener Wirkstoff] | 37,4 | 25,3 |
| FPF nach 7 Tagen bei 40°C/75% r.F. [% gefundener Wirkstoff] | 35,6 | 9,7 |

### Beispiel 4

1196 g Lactose-Monohydrat mit einer definierten Korngrösse < 315 µm für 100%, < 150 µm für 55-90% und < 63 µm für maximal 10% der Partikel (Siebanalyse) werden gesiebt und mit 3 g gesiebtem Magnesiumsstearat in einem Taumelmischer (tumble blender TB) gemischt. Im Anschluss werden 1,44 g Formoterolfumarat-Dihydrat und die Vormischung gesiebt und gemischt. In analoger Weise werden unter Variation der Ansatzgrösse, der Prozessparameter und der Magnesiumstearat- und Formoterolfumarat-Mengen weitere Formulierungen hergestellt, um deren Einfluss auf die Stabilität der FPD zu untersuchen. Die erhaltenen Mischungen werden - nach Herstellung oder nach anschliessender Lagerung der offenen Mischung bei erhöhter Temperatur und Feuchtigkeit - in einen geeigneten dosierenden Trockenpulverinhalator gefüllt. Die in-vitro Partikelgrössenverteilung und die FPD bzw. FPF werden mit einem Multi-Stage Liquid Impinger an einer adäquaten Anzahl Dosierungen bestimmt.

Die Ergebnisse zeigten, dass bei Herstellung der Pulvermischungen mit einem Taumelmischer praktisch nur die Konzentration von Magnesiumstearat für die Stablilität hinsichtlich der FPD verantwortlich ist, während die anderen Parameter im untersuchten Bereich praktisch ohne Bedeutung für die Stabilität bei erhöhter Feuchtigkeit waren. In Tabelle 4 sind die Ansatzgrösse, die Konzentration an Magnesiumstearat (MS) und die Konzentration an Formoterolfumarat-Dihydrat (FF) für einige repräsentative Mischungen sowie deren in einem 5-Stufen Liquid Impinger gemäss Ph. Eur. gemessene FPF-Werte, die unmittelbar nach Herstellung bzw. nach Lagerung bei 40°C / 75% r.F. während 7 Tagen erhalten wurden, zusammengestellt. Die angegebenen Werte sind Mittelwerte aus je drei Bestimmungen. Die Ergebnisse zeigen, dass die FPF bei ausreichender Magnesiumstearatkonzentration durch erhöhte Temperatur und Feuchtigkeit kaum mehr beeinträchtigt wird. Die für Formulierung 1-A nach 3 Wochen Lagerung bei 40°C / 75% r.F. gemessene FPF von 32,3% scheint zudem anzudeuten, dass selbst bei suboptimaler Magnesiumstearatkonzentration ein langanhaltender Schutz gegen den Einfluss erhöhter Temperatur und Feuchtigkeit erreicht wird.

**Tabelle 4**

| Formulierung | Ansatzgrösse | MS | FF | FPF nach 0 d | FPF nach 7 d |
|---|---|---|---|---|---|
| | [kg] | [%G/G] | [%G/G] | [%] | [%] |
| 4-A | 1,2 | 0,25 | 0,12 | 42,5 | 33,6 |
| 4-B | 4,8 | 0,50 | 0,12 | 49,3 | n.b. |
| 4-C | 4,8 | 0,75 | 0,12 | 56,9 | 56,8 |
| 4-D | 1,2 | 0,25 | 0,34 | 50,0 | 33,5 |
| 4-E | 4,8 | 0,50 | 0,34 | 58,1 | n.b. |
| 4-F | 4,8 | 0,75 | 0,34 | 59,2 | 57,2 |
| 1-C (Vergleich) | 0,2 | 0,00 | 0,27 | 39,7 | 12,3 |
| 1-A | 0,2 | 0,50 | 0,27 | 44,8 | 32,5 |

### Beispiel 5

49,5 g Lactose-Monohydrat mit einer definierten Korngrösse < 200 µm für 100%, < 125 µm für 50% und < 75 µm für 10% der Partikel (Siebanalyse) werden gesiebt und mit 0,25 g gesiebtem Magnesiumstearat in einem Taumelmischer gemischt. Im Anschluss werden 0,25 g Salbutamolsulfat und die Vormischung gesiebt und gemischt. In analoger Weise werden unter Variation der Konzentration an Magnesiumstearat (MS) und Salbutamolsulfat (SS) weitere Mischungen gemäss Tabelle 5 hergestellt. Die erhaltenen Mischungen werden unmittelbar nach Herstellung oder nach Lagerung bei 40°C / 75% r.F. während 5 bzw. 21 Tagen in einen geeigneten dosierenden Trockenpulverinhalator gefüllt. Zur Bestimmung der FPD bzw. FPF wird eine adäquate Anzahl Dosierungen in einen Twin Impinger gemäss Ph. Eur. abgegeben, gesammelt und der Wirkstoffgehalt der einzelnen Fraktionen analytisch bestimmt.

Die in Tabelle 5 angegebenen FPF-Werte (Mittelwerte aus zwei Messungen) zeigen, dass Magnesiumstearat auch im Falle des feuchtigkeitsempfindlichen Wirkstoffes Salbutamolsulfat einen Schutz gegen erhöhte Temperatur und Feuchtigkeit bewirkt, aber eine Stabilisierung der FPF erst bei höheren Magnesiumstearat-Konzentrationen erreicht wird als bei den Formoterolfumarat-Zubereitungen.

**Tabelle 5**

| Formulierung | MS | SS | FPF [%] nach | | |
|---|---|---|---|---|---|
| | [%G/G] | [%G/G] | 0 d | 5 d | 21 d |
| 5-A | 0,5 | 0,5 | 9,3 | 14,2 | 12,0 |
| 5-B | 0,5 | 1,0 | 22,3 | 17,1 | 14,9 |
| 5-C | 0,5 | 2,5 | 30,2 | 25,6 | 22,3 |
| 5-D | 1,0 | 0,5 | 19,0 | 18,8 | 13,5 |
| 5-E | 1,0 | 1,0 | 23,0 | 20,1 | 15,8 |
| 5-F | 1,0 | 2,5 | 25,0 | 22,6 | 20,8 |
| 5-G | 2,5 | 1,0 | 22,7 | 23,4 | 21,5 |
| 5-H | 2,5 | 2,5 | 25,9 | 26,4 | 27,4 |

| Vergleich: | | | | | |
|---|---|---|---|---|---|
| 5-I | 0,0 | 0,5 | 13,5 | 5,3 | 4,0 |
| 5-J | 0,0 | 1,0 | 19,7 | 9,5 | 6,6 |
| 5-K | 0,0 | 2,5 | 25,3 | 14,8 | 13,9 |

### Beispiel 6

99,2 g Lactose-Monohydrat mit einer Korngrösse < 315 µm für 100%, < 150 µm für 55-90% und < 63 µm für maximal 10% der Partikel (Siebanalyse) werden gesiebt und mit 0,5 g gesiebtem Magnesiumstearat in einem Taumelmischer gemischt. Im Anschluss werden 0,34 g Tiotropiumbromid und die Vormischung gesiebt und gemischt. Die erhaltene Mischung wird nach der Herstellung bzw. nach Lagerung bei 40°C / 75% r.F. während 7 Tagen in einen geeigneten dosierenden Trockenpulverinhalator gefüllt. Zur Bestimmung der FPD bzw. FPF wird eine adäquate Anzahl Dosierungen in einen Multi-Stage Impinger gemäss Ph. Eur. abgegeben, gesammelt und der Wirkstoffgehalt der einzelnen Fraktionen analytisch bestimmt. Die unmittelbar nach Herstellung abgefüllten Proben ergaben eine FPD von 8,0 µg und eine FPF von 48,4% (Mittelwerte aus 2 Messungen); für die während 7 Tagen unter feuchten Bedingungen gelagerten Proben wurden eine FPD von 6,9 µg und eine FPF von 43,0% erhalten (Mittelwerte aus 4 Messungen), d.h. die Stabilisierung mit 0,5% Magnesiumstearat ergibt auch im Falle des feuchtigkeitsempfindlichen Tiotropiumbromids eine hinreichend gleichmässige FPD bzw. FPF.

### Beispiel 7

Zur Untersuchung des Einflusses erhöhter Feuchtigkeit und Temperatur auf erfindungsgemässe Formulierungen unter praxisnahen Bedingungen wurden Trockenpulverinhalatoren vom Typ SkyePharma mDPI (SkyePharma AG, Schweiz), entsprechend der Offenbarung von WO-A-97/20589, mit je 2 g gemäss Beispiel 1 frisch hergestelltem Trockenpulver aus 99,23 Gew.-% Lactose-Monohydrat, 0,50 Gew.-% Magnesiumstearat und 0,27 Gew.-% mikronisiertem Formoterolfumarat-Dihydrat (Formulierung 1-A) gefüllt. Die in-vitro-Daten wurden unmittelbar nach dem Füllen sowie nach 3, 6 und 12 Monaten Lagerung der unverpackten Inhalatoren ohne Feuchtigkeitsschutz bei verschiedenen Temperatur- und Feuchtebedingungen ermittelt. Die abgegebenen Dosen und die Hubmassen wurden anhand der Hübe Nr. 2-4, 149-152 und 298-300 aus je drei Inhalatoren bestimmt, die gemäss der von Collins auf der Konferenz Drug Delivery to the Lungs VIII, London, Dezember 1998 (Tagungsunterlagen Seiten 116-119) beschriebenen Methode in einen Buchner-Trichter abgegeben wurden. Die FPD bzw. FPF wurde bei 60 1/min. mittels eines 5-Stufen Liquid Impactors gemäss Ph. Eur. anhand der Hübe Nr. 6-15 und 287-296 aus je drei Inhalatoren bestimmt. Die in Tabelle 6 zusammengestellten Mittelwerte und relativen Standardabweichungen zeigen, dass die erfindungsgemässe Formulierung selbst bei vergleichsweise extremen Temperatur- und Feuchtebedingungen über lange Zeiträume kaum beeinträchtigt wird.

**Tabelle 6**

| Lagerung | Hubmasse | Abgegebene | FPF | FPD |
|---|---|---|---|---|
| | [mg] | Dosis [µg] | [%] | [µg] |
| nein | 6,0 (± 5,4%) | 10,2 (± 10,1%) | 43,5 | 6,0 |

| 25°C / 60% r.F.: | | | | |
|---|---|---|---|---|
| 3 Monate | 6,1 (± 4,8%) | 10,5 (± 9,5%) | 40,8 | 5,4 |
| 6 Monate | 5,9 (± 8,2%) | 10,9 (± 6, 9%) | 47,8 | 7,0 |
| 12 Monate | 6,1 (± 5, 0%) | 12,1 (± 5,9%) | 42,2 | 5, 9 |

| 30°C / 70% r.F.: | | | | |
|---|---|---|---|---|
| 3 Monate | 6,1 (± 6, 9%) | 11,0 (± 12, 9%) | 40,1 | 5,6 |
| 6 Monate | 6,2 (± 8,7%) | 10,6 (± 11,5%) | 39,9 | 5,7 |
| 12 Monate | 6,3 (± 4,3%) | 10,7 (± 5,9%) | 42,0 | 5,7 |

| 40°C / 75% r.F.: | | | | |
|---|---|---|---|---|
| 3 Monate | 5,8 (± 9,7%) | 9,9 (± 9,8%) | 38,1 | 5,2 |
| 6 Monate | 6,0 (± 19,5%) | 10,3 (± 19,2%) | 35,1 | 4,9 |
| 12 Monate | 6,7 (± 6,8%) | 10,7 (± 7,9%) | 37,9 | 5,4 |

### Beispiel 8

In analoger Weise zu Beispiel 4 wurde ein Trockenpulver, bestehend aus 0,2 Gew.-% Formoterolfumarat-Dihydrat, 0,5 Gew.-% Glycopyrrolate, 0,5 Gew.-% Magnesiumstearat und 98,8 Gew.-% Lactose-Monohydrat hergestellt.

## Patentansprüche

1. Trockenpulver-Formulierung zur Inhalation, die ein Pulvergemisch enthaltend einen pharmazeutisch nicht wirksamen Träger in nicht inhalierbarer Teilchengrösse, einen fein verteilten pharmazeutischen Wirkstoff in inhalierbarer Teilchengrösse und staubförmiges Magnesiumstearat einer mittleren Teilchengrösse von 1 bis 100 µm umfasst.

2. Trockenpulver-Formulierung gemäss Anspruch 1, wobei das Magnesiumstearat eine mittlere Teilchengrösse von 5 bis 20 µm hat.

3. Trockenpulver-Formulierung gemäss Anspruch 1 oder 2, worin der Wirkstoff und das Magnesiumstearat durch Adhäsion an die Trägerteilchen gebunden sind.

4. Trockenpulver-Formulierung gemäss einem der Ansprüche 1 bis 3, worin das Magnesiumstearat in einer Konzentration von 0,1 bis 2 Gew.-%, bezogen auf die Gesamtformulierung, vorliegt.

5. Trockenpulver-Formulierung gemäss einem der Ansprüche 1 bis 4, worin das Magnesiumstearat in einer Konzentration von 0,25 bis 1 Gew.-%, vorzugsweise 0,4 bis 0,8 Gew.-%, bezogen auf die Gesamtformulierung, vorliegt.

6. Trockenpulver-Formulierung gemäss einem der Ansprüche 1 bis 5, wobei die Trockenpulver-Formulierung einen pharmazeutischen Wirkstoff enthält, der in Form eines pharmazeutisch annehmbaren Salzes oder Esters vorliegt.

7. Trockenpulver-Formulierung gemäss einem der Ansprüche 1 bis 6, wobei die Trockenpulver-Formulierung einen pharmazeutischen Wirkstoff enthält, der in Form eines pharmazeutisch annehmbaren Salzes oder Esters vorliegt, und das Magnersiumstearat in einer Konzentration von 0.25 bis 1 Gew.-%, bezogen auf die Gesamtformulierung, vorliegt.

8. Trockenpulver-Formulierung gemäss einem der Ansprüche 1 bis 7, wobei die Trockenpulver-Formulierung als pharmazeutischen Wirkstoff ein Betamimetikum, ein Anticholinergikum, ein Corticosteroid, einen Leukorienantagonist, einen Phosphodiesterase-Inhibitor, einen PAF-Inhibitor, einen Kaliumkanalöffner, ein Schmerzmittel, ein Potenzmittel, ein Peptid oder ein Protein enthält.

9. Trockenpulver-Formulierung gemäss einem der Ansprüche 1 bis 8, wobei die Trockenpulver-Formulierung als pharmazeutischen Wirkstoff ein Betamimetikum aus der Gruppe, umfassend Levalbuterol, Terbutalin, Reproterol, Salbutamol, Salmeterol, Formoterol, Fenoterol, Clenbuterol, Bambuterol, Tulobuterol, Broxaterol, Epinephrin, Isoprenalin und Hexoprenalin, ein Anticholinergikum aus der Gruppe, umfassend Tiotropium, Ipratropium, Oxitropium und Glycopyrronium, ein Corticosteroid aus der Gruppe, umfassend Butixocart, Rofleponid, Budesonid, Ciclosenid, Mometason, Fluticason, Beclomethason, Loteprednol und Triamcinolon, einen Leukotrienantagonisten aus der Gruppe, umfassend Andolast, Iralukast, Pranlukast, Imitrodast, Seratrodast, Zileuton, Zafirlukast und Montelukast, einen Phosphodiesterase-Inhibitor aus der Gruppe, umfassend Filaminast und Piclamilast, einen PAF-Inhibitor aus der Gruppe, umfassend Apafant, Forapafant und Israpafant, einen Kaliumkanalöffner aus der Gruppe, umfassend Amilorid und Furosemid, ein Schmerzmittel aus der Gruppe, umfassend Morphin, Fentanyl, Pentazozin, Buprenorphin, Pethidin, Tilidin, Methadon und Heroin, ein Potenzmittel aus der Gruppe, umfassend Sildenafil, Alprostadil und Phentolamin, ein Peptid oder Protein aus der Gruppe, umfassend Insulin, Erythropoietin, Gonadotropin und Vasopressin, oder ein pharmazeutisch annehmbares Derivat oder Salz einer dieser Verbindungen enthält.

10. Trockenpulver-Formulierung gemäss einem der Ansprüche 1 bis 9, wobei die Trockenpulver-Formulierung als pharmazeutischen Wirkstoff ein Betamimetikum aus der Gruppe, umfassend Salbutamol, Salmeterol, Formoterol, Fenoterol, Clenbuterol, Bambuterol, Tulobuterol, Broxaterol, Epinephrin, Isoprenalin und Hexoprenalin, ein Anticholinergikum aus der Gruppe, umfassend Tiotropium, Ipratropium, Oxitropium und Glycopyrronium, ein Corticosteroid aus der Gruppe, umfassend Budesonid, Ciclosenid, Mometason, Fluticason, Beclomethason, Loteprednol und Triamcinolon, einen Leukotrienantagonisten aus der Gruppe, umfassend Zileuton, Zafirlukast und Montelukast, einen Kaliumkanalöffner aus der Gruppe, umfassend Amilorid und Furosemid, ein Schmerzmittel aus der Gruppe, umfassend Morphin, Fentanyl, Pentazozin, Buprenorphin, Pethidin, Tilidin, Methadon und Heroin, ein Potenzmittel aus der Gruppe, umfassend Sildenafil, Alprostadil und Phentolamin, ein Peptid oder Protein aus der Gruppe, umfassend Insulin, Erythropoietin, Gonadotropin und Vasopressin, oder ein pharmazeutisch annehmbares Derivat oder Salz einer dieser Verbindungen enthält.

11. Trockenpulver-Formulierung gemäss einem der Ansprüche 1 bis 10, wobei die Trockenpulver-Formulierung als pharmazeutischen Wirkstoff ein Betamimetikum und/oder ein Anticholinergikum und/oder ein Corticosteroid enthält, das vorzugsweise in Form eines pharmazeutisch annehmbaren Salzes oder Esters vorliegt.

12. Trockenpulver-Formulierung gemäss einem der Ansprüche 1 bis 11, wobei die Trockenpulver-Formulierung als pharmazeutischen Wirkstoff ein Betamimetikum aus der Gruppe, umfassend Levalbuterolsulfat, Formoterolfumarat, Formoteroltartrat, Salbutamolsulfat und Salmeterolxinafoat, und/oder ein Anticholinergikum aus der Gruppe, umfassend Oxitropiumbromid, Glycopyrrolat, Ipratropiumbromid und Tiotropiumbromid, enthält.

13. Trockenpulver-Formulierung gemäss einem der Ansprüche 1 bis 12, wobei die Trockenpulver-Formulierung als pharmazeutischen Wirkstoff Formoterol oder ein pharmazeutisch annehmbares Salz davon enthält.

14. Trockenpulver-Formulierung gemäss einem der Ansprüche 1 bis 13, wobei die Trockenpulver-Formulierung als pharmazeutischen Wirkstoff ein Corticosteroid aus der Gruppe, umfassend Beclomethason-dipropiponat, Fluticason-propionat, Triamcinolon-16,21-diacetat, Triamcinolonacetonid-21-acetat, Triamcinolonacetonid-21-dinatriumphosphat, Triamcinolonacetonid-21-hemisuccinat, Mometasonfuroat und Loteprednol-etabonat, enthält.

15. Trockenpulver-Formulierung gemäss einem der Ansprüche 1 bis 14, wobei die Trockenpulver-Formulierung als pharmazeutischen Wirkstoff ein Corticosteroid in Kombination mit einem Betamimetikum, vorzugsweise ein Corticosteroid aus der Gruppe, umfassend Ciclosenid, Rofleponid, Fluticason-propionat, Mometasonfuroat und Loteprednol-etabonat, in Kombination mit einem Betamimetikum aus der Gruppe, umfassend Formoterolfumarat, Formoteroltartrat, Levalbuterolsulfat und Salmeterolxinafoat, enthält.

16. Trockenpulver-Formulierung gemäss einem der Ansprüche 1 bis 15, wobei die Trockenpulver-Formulierung als Träger ein Mono- oder Disaccharid, einen Zuckeralkohol, Polymilchsäure oder Cyclodetrin enthält.

17. Trockenpulver-Formulierung gemäss einem der Ansprüche 1 bis 16, wobei die Trockenpulver-Formulierung als Träger Glucose, Lactose-Monohydrat oder Trehalose enthält.

18. Trockenpulver-Formulierung gemäss einem der Ansprüche 1 bis 17, worin die Trockenpulver-Formulierung zusätzlich einen Anteil an Trägerpartikeln in inhalierbarer Teilchengrösse enthält.

19. Verfahren zur Herstellung einer Trockenpulver-Formulierung, wobei einen pharmazeutisch nicht wirksamen Träger in nicht inhalierbarer Teilchengrösse, einen fein verteilten pharmazeutischen Wirkstoff in inhalierbarer Teilchengrösse und staubförmiges Magnesiumstearat einer mittleren Teilchengrösse von 1 bis 100 µm miteinander vermischt.

20. Verfahren gemäss Anspruch 19, wobei eine Vormischung von Magnesiumstearat mit dem Träger hergestellt und dann der Wirkstoff zugemischt wird.

21. Verfahren gemäss Anspruch 19, wobei eine Vormischung des Wirkstoffes mit dem Träger hergestellt und dann das Magnesiumstearat zugemischt wird.
